# EUROPEAN PATENT APPLICATION

(11) **EP 4 316 484 A1**
(43) Date of publication of application: **07.02.2024**
(21) Application number: 22775354.8
(22) Date of filing: 16.03.2022
(51) Int. Cl.: A61K 31/23, A23L 33/115, A61K 31/231, A61P 3/00, A61P 3/04, A61P 3/10

(54) **METHOD FOR RAISING BLOOD DECANOIC ACID CONCENTRATION, BLOOD-DECANOIC-ACID-CONCENTRATION-RAISING AGENT, PHARMACEUTICAL COMPOSITION, AND FOOD COMPOSITION**

(30) Priority: 26.03.2021 JP 2021053119
(71) Applicant: THE NISSHIN OILLIO GROUP, LTD., Tokyo 104-8285 (JP)
(72) Inventor: TSUJINO Shogo, Yokohama-shi, Kanagawa 235-8558 (JP); TAKAGI Tetsuo, Yokohama-shi, Kanagawa 235-8558 (JP)
(74) Representative: KATZAROV S.A.
(86) International application number: PCT/JP2022/012042
(87) International publication number: WO 2022/202566

(57) **Abstract**

The present invention addresses the problem of providing a technique with which it is possible to increase the decanoic acid concentration in the peripheral blood and to deliver decanoic acid effective for manifesting a physiological action in the peripheral tissues without a large amount of MCT being ingested at one time. The present invention is: a method for increasing the blood decanoic acid concentration through ingestion of a triglyceride having, as constituent fatty acids, decanoic acid and long-chain fatty acids; a blood-decanoic-acid-concentration- increasing agent in which a triglyceride having, as constituent fatty acids, decanoic acid and long-chain fatty acids is employed as an active ingredient; and a pharmaceutical composition and a food composition that contain the blood-decanoic-acid-concentration- increasing agent.

## Description

### TECHNICAL FIELD

The present invention relates to a method for increasing a blood decanoic acid concentration, a blood-decanoic-acid-concentration-increasing agent, a pharmaceutical composition and a food composition.

### BACKGROUND ART

When medium chain fatty acids having 6 to 12 carbon atoms are ingested, they are commonly ingested as medium chain fatty acid triglycerides having ester-bonds with glycerin (hereinafter, also referred to as MCT). It is known that an MCT is ingested and then quickly oxidized in the liver to form a ketone body, which is delivered to various organs through the blood. The ketone body is an energy source for mitochondria in cells and an energy source for nerve cells, and thus has been utilized for e.g. epilepsy and Alzheimer's disease.

As described above, it has been so far considered that many physiological functions exhibited by an MCT are exerted via the ketone body; however, it has been reported in recent years that the medium chain fatty acid having 10 carbon atoms (hereinafter, also referred to as decanoic acid) enhances the lipid oxidizing ability of skeletal muscles (Non-Patent Document 1). It has been also reported that the medium chain fatty acid having 10 carbon atoms enhances glucose-responsive insulin secretion in pancreatic β cells (Non-Patent Document 2). It has been further reported that a receptor (GPR84: G protein-coupled receptor 84) that contains medium chain fatty acids having 10 to 12 carbon atoms as ligands is expressed in various organs (Non-Patent Document 3), and that an effect of obtaining a constitution that body fat is easily burnt is expected by increasing mitochondria in skeletal muscles via the receptor (Non-Patent Document 4).

Generally, the ingested MCT is digested and absorbed, and then mostly metabolized to a ketone body in the liver through portal veins. Therefore, it has been difficult to deliver medium chain fatty acids such as decanoic acid to peripheral tissues due to the characteristics of digestion and absorption (that is, to increase the concentration of the medium chain fatty acids in the peripheral blood). In addition, it is required to ingest a large amount of MCT at one time to increase the concentration of medium chain fatty acids in the peripheral blood, and there has been also a problem that gastric distress is caused by ingestion.

### Citation List

### Patent Document

Non-Patent Document 1: Journal of Nutritional Science and Vitaminology, 62, 32-39 (2016)
Non-Patent Document 2: Nutrients, 10, 473 (2018)
Non-Patent Document 3: Journal of Biological Chemistry, 281, 34457-34464 (2006)
Non-Patent Document 4: The FASEB Journal, 33, 12264-12276 (2019)

### DISCLOSURE OF THE INVENTION

### Problems to be Solved by the Invention

In view of the above problems, an object of the present invention is to provide a technique that can increase a decanoic acid concentration in the peripheral blood and deliver decanoic acid that is effective to express the physiological action to peripheral tissues, without ingesting a large amount of decanoic acid at one time.

### Means for Solving the Problems

The present inventors unexpectedly found that the decanoic acid concentration in the peripheral blood could be significantly increased by ingestion of a triglyceride, having decanoic acid and a long chain fatty acid as constituent fatty acids, thereby completing the present invention. Specifically, the present invention provides the following.

(1) A method for increasing a blood decanoic acid concentration by ingestion of a triglyceride having decanoic acid and a long chain fatty acid as constituent fatty acids.
(2) The method for increasing a blood decanoic acid concentration according to (1), wherein the triglyceride includes a triglyceride in which two of the constituent fatty acids are decanoic acid and one is a long chain fatty acid, in a content of 50 mass% or more.
(3) The method for increasing a blood decanoic acid concentration according to (1) or (2), wherein the long chain fatty acid is an unsaturated fatty acid.
(4) The method for increasing a blood decanoic acid concentration according to (3), wherein the unsaturated fatty acid is oleic acid.
(5) The method for increasing a blood decanoic acid concentration according to any one of (1) to (4), wherein slim body shape, prevention of obesity, or improvement in obesity is promoted by increasing the blood decanoic acid concentration.
(6) The method for increasing a blood decanoic acid concentration according to any one of (1) to (4), wherein postprandial elevation of blood glucose level is suppressed and diabetes is treated or prevented by increasing a blood decanoic acid concentration.
(7) A blood-decanoic-acid-concentration-increasing agent, including a triglyceride as an active ingredient, the triglyceride having decanoic acid and a long chain fatty acid as constituent fatty acids.
(8) The blood-decanoic-acid-concentration-increasing agent according to (7), wherein the triglyceride includes a triglyceride in which two of the constituent fatty acids are decanoic acid and one is a long chain fatty acid, in a content of 50 mass% or more.
(9) The blood-decanoic-acid-concentration-increasing agent according to (7) or (8), wherein the long chain fatty acid is an unsaturated fatty acid.
(10) The blood-decanoic-acid-concentration-increasing agent according to (9), wherein the unsaturated fatty acid is oleic acid.
(11) A pharmaceutical composition for use in treatment or prevention of obesity or diabetes, the pharmaceutical composition including the blood-decanoic-acid-concentration-increasing agent according to any one of (7) to (10).
(12) A food composition for use in any one of suppression of postprandial elevation of blood glucose level, slim body shape, prevention of obesity, or improvement in obesity, the food composition including the blood-decanoic-acid-concentration-increasing agent according to any one of (7) to (10) .
(13) Use of a triglyceride having decanoic acid and a long chain fatty acid as constituent fatty acids for manufacturing a blood-decanoic-acid-concentration-increasing agent.

### Effects of the Invention

According to the present invention, there is provided a technique which can obtain a higher decanoic acid concentration in the peripheral blood by ingestion of a triglyceride, having as constituent fatty acids decanoic acid and a long chain fatty acid, than the concentration in a method by ingestion of only a medium chain fatty acid triglyceride having as constituent fatty acids decanoic acid.

### BRIEF DESCRIPTION OF THE DRAWINGS

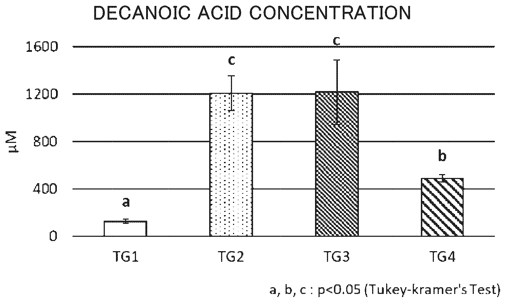

Fig. 1 is a graph showing the decanoic acid concentration in the peripheral blood by the method for increasing a blood decanoic acid concentration of the present invention.

### PREFERRED MODE FOR CARRYING OUT THE INVENTION

Specific embodiments of the present invention will now be described in detail. It should be noted, however, that the present invention is not limited to the following embodiments in any way. The present invention can be implemented with appropriate modifications without departing from the object of the present invention. It should be noted that e.g. preferred aspects and more preferred aspects described below as examples can be used in an appropriate combination regardless of the expressions of e.g. "preferred" and "more preferred." In addition, value ranges are described as examples, and ranges in which the upper limit and the lower limit of each range, and values in Examples are appropriately combined can be also preferably used regardless of the expressions of e.g. "preferred" and "more preferred." Furthermore, a term of e.g. "contain" or "include" can be appropriately read as "essentially consist of" and "consist of."

### [Method for increasing blood decanoic acid concentration]

The method for increasing a blood decanoic acid concentration of the present invention is a method for increasing a decanoic acid concentration in the peripheral blood to the concentration at which the physiological action is expressed by ingestion of a triglyceride, having as constituent fatty acids decanoic acid and a long chain fatty acid. In addition, in the method for increasing a blood decanoic acid concentration of the present invention, the maximum peak concentration in the peripheral blood is preferably raised twice or higher than the concentration when ingesting a triglyceride, having as constituent fatty acids only decanoic acid (hereinafter also referred to as tridecanoin), so that the amount of decanoic acid ingested will be the same.

According to the method for increasing a blood decanoic acid concentration of the present invention, the effect of increasing a decanoic acid concentration in the peripheral blood is more excellent than that when ingesting tridecanoin. Therefore, a desired blood decanoic acid concentration can be obtained by ingesting a lower amount of the triglyceride than the amount when ingesting tridecanoin, and moreover gastric distress caused when ingesting a large amount of tridecanoin can be reduced.

In the present invention, "an increase in the blood decanoic acid concentration" indicates that the decanoic acid concentration in the peripheral blood is raised to an extent in which the desired physiological action is expressed. The decanoic acid concentration in the peripheral blood is not particularly limited as long as the desired physiological action is expressed. In the case of human, for example, the concentration is preferably 0.5 to 2000 µM, more preferably 1 to 1000 µM, yet further preferably 5 to 500 µM, and most preferably 10 to 200 µM. The decanoic acid concentration in the peripheral blood can be measured by a gas chromatography method about the total lipid fraction in serum. In the present invention, the method for ingesting a triglyceride, having as constituent fatty acids decanoic acid and a long chain fatty acid, is preferably oral ingestion.

It is thought that by the method for increasing a blood decanoic acid concentration of the present invention, decanoic acid in the peripheral blood is not metabolized to the ketone body, reaches skeletal muscle cells in the state of decanoic acid, acts directly and/or as a ligand of GPR84, and can enhance an increase in skeletal muscle mitochondria and/or the lipid oxidizing ability of skeletal muscles. Therefore, the effect of obtaining a constitution which easily burns body fat is expected, and thus the effects of obesity prevention or improvement and slim body shape by body fat reduction and/or suppression of body fat accumulation can be expected.

It is also thought that by the method for increasing a blood decanoic acid concentration of the present invention, decanoic acid in the peripheral blood is not metabolized to the ketone body, reaches pancreatic β cells in the state of decanoic acid, acts, and can enhance glucose-responsive insulin secretion. Therefore, the effect of suppressing postprandial elevation of blood glucose level, and the effect of diabetes treatment or prevention can be expected.

The method for increasing a blood decanoic acid concentration of the present invention can be expected to strengthen and enhance a physiological effect expressed via GPR84 in addition to the above physiological effect.

### [Triglyceride having as constituent fatty acids decanoic acid and a long chain fatty acid]

The triglyceride having decanoic acid and a long chain fatty acid as constituent fatty acids in the present invention is a triglyceride having one or two decanoic acids ester-bound in a triglyceride molecule. The triglyceride specifically indicates a triglyceride including one or two selected from a triglyceride having one decanoic acid and two long chain fatty acids ester-bound to a triglyceride molecule (hereinafter also referred to as DL2), and a triglyceride having two decanoic acids and one long chain fatty acid ester-bound to a triglyceride molecule (hereinafter also referred to as D2L). The long chain fatty acids bound to DL2 may be different long chain fatty acids. It should be noted that decanoic acid in the present invention is n-decanoic acid.

The long chain fatty acids are fatty acids having 14 to 24 carbon atoms, and examples thereof include saturated long chain fatty acids such as myristic acid, palmitic acid, stearic acid, arachidic acid, behenic acid and lignoceric acid, and unsaturated long chain fatty acids such as palmitoleic acid, oleic acid, linoleic acid, linolenic acid, γ-linolenic acid, arachidonic acid and icosapentaenoic acid. In addition, the long chain fatty acid is preferably an unsaturated fatty acid and more preferably oleic acid in that the effect of the present invention is easily achieved. In long chain fatty acids in all the constituent fatty acids of DL2 and/or D2L, the proportion of unsaturated fatty acids to the long chain fatty acids is preferably 70 to 100 mass%, more preferably 80 to 98 mass%, and most preferably 90 to 96 mass%. In addition, in long chain fatty acids in all the constituent fatty acids of DL2 and/or D2L, the proportion of oleic acid to the long chain fatty acids is preferably 70 to 100 mass%, more preferably 80 to 98 mass%, and most preferably 90 to 96 mass%. When the proportion of unsaturated fatty acids or oleic acid is within the above range, the effect of the present invention is easily achieved.

The triglyceride, having as constituent fatty acids decanoic acid and a long chain fatty acid, in the present invention contains, in the sum of the triglyceride, preferably 50 mass% or more, more preferably 50 to 100 mass%, yet further preferably 70 to 100 mass% and most preferably 85 to 100 mass% of a triglyceride in which two of the constituent fatty acids are decanoic acid and one is a long chain fatty acid (D2L). When the amount of D2L is within the above range, because the amount of decanoic acid ingested is increased, the effect of increasing a blood decanoic acid concentration can be further obtained.

Examples of the method for confirming and determining the molecular species (DL2 and/or D2L) in the triglyceride, having as constituent fatty acids decanoic acid and a long chain fatty acid, in the present invention include a method using gas chromatography (JAOCS, vol 70, 11, 1111-1114 (1993) and the silver ion column-HPLC method (in accordance with J. High Resol. Chromatogr., 18, 105-107 (1995)). In addition, examples of the method for confirming and determining decanoic acid and long chain fatty acids in the triglyceride include a method for quantitative analysis in accordance with "Standard Method for the Analysis of Fats, Oils and Related Materials 2.4.2.3-2013 fatty acid compositional ratio (capillary gas chromatography)" established by Japan Oil Chemists' Society.

The method for producing the triglyceride, having as constituent fatty acids decanoic acid and a long chain fatty acid, in the present invention is not particularly limited, and examples thereof include a production method by a conventionally known esterification reaction or transesterification reaction using as raw materials glycerin, decanoic acid, tridecanoin, a long chain fatty acid triglyceride (hereinafter also referred to as LCT) and the like. In the esterification reaction, the triglyceride can be produced, for example, by heating and dehydration condensation of fatty acids and glycerin under reduced pressure. Examples of the transesterification reaction include chemical transesterification reaction using an inorganic catalyst such as sodium methoxide, and transesterification reaction using an enzyme such as lipase. The transesterification reaction may be selective transesterification reaction or non-selective transesterification reaction. When D2L is produced, for example, decanoic acid and LCT are transesterified using an sn-1,3-position specific enzyme to obtain a reactant containing a triglyceride having as constituent fatty acids decanoic acid and a long chain fatty acid. Next, the obtained reactant is purified to obtain D2L in which decanoic acid is at the sn-1,3 positions in the triglyceride and a long chain fatty acid is at the sn-2 position.

### [Blood-decanoic-acid-concentration-increasing agent]

The blood-decanoic-acid-concentration-increasing agent of the present invention includes a triglyceride, having as constituent fatty acids decanoic acid and a long chain fatty acid, as an active ingredient. By ingesting the blood-decanoic-acid-concentration-increasing agent of the present invention, the decanoic acid concentration in the peripheral blood can be raised to the concentration at which the physiological action is expressed. The blood-decanoic-acid-concentration-increasing agent of the present invention has a more excellent effect of increasing a decanoic acid concentration in the peripheral blood than that when ingesting tridecanoin. Therefore, a desired blood decanoic acid concentration can be obtained by ingesting a lower amount of the triglyceride than the amount when ingesting tridecanoin, and moreover gastric distress caused when ingesting a large amount of tridecanoin can be reduced.

The blood-decanoic-acid-concentration-increasing agent of the present invention may contain triglyceride molecular species other than DL2 and D2L as long as the effect of the present invention is not inhibited. Examples of the triglyceride molecular species include MCT, LCT and the like. In addition, the blood-decanoic-acid-concentration-increasing agent of the present invention may also contain fats and oils including the above triglyceride molecular species. The fats and oils are not particularly limited as long as they are those used as food, and are, for example, soybean oil, high oleic acid soybean oil, rapeseed oil, high oleic acid rapeseed oil, corn oil, sesame oil, sesame salad oil, shiso oil, linseed oil, peanut oil, safflower oil, high oleic acid safflower oil, sunflower oil, high oleic acid sunflower oil, cottonseed oil, grapeseed oil, macadamia nut oil, hazelnut oil, pumpkin seed oil, walnut oil, camellia oil, tea seed oil, perilla oil, borage oil, olive oil, rice bran oil, wheat germ oil, palm oil, palm kernel oil, coconut oil, cacao butter, beef tallow, lard, chicken fat, milk fat, fish oil, seal oil, algae oil and the like. They are also transesterified fats and oils obtained by transesterifying two or more of these fats and oils, hydrogenated fats and oils of these fats and oils, separated fats and oils of these fats and oils and the like. The above fats and oils may be used individually or two or more of them may be used in combination.

The blood-decanoic-acid-concentration-increasing agent of the present invention contains, in all triglycerides, preferably 50 to 100 mass%, more preferably 70 to 95 mass% and most preferably 80 to 90 mass% of DL2 and/or D2L, an active ingredient. In addition, the DL2 and/or D2L contains, in the sum of the triglycerides, preferably 50 mass% or more, more preferably 50 to 100 mass%, yet further preferably 70 to 100 mass% and most preferably 85 to 100 mass% of D2L. When the amount of D2L is within the above range, because the amount of decanoic acid ingested is increased, the effect of increasing a blood decanoic acid concentration can be further obtained.

The method for producing DL2 and/or D2L, an active ingredient, of the blood-decanoic-acid-concentration-increasing agent of the present invention, the effective blood concentration to express a desired physiological activity, and health functions and pharmacological effects expected are the same as in the method for increasing a blood decanoic acid concentration of the present invention.

### [Pharmaceutical composition]

The blood-decanoic-acid-concentration-increasing agent of the present invention can be applied to produce a pharmaceutical composition used to treat or prevent obesity or diabetes. The pharmaceutical composition including the blood-decanoic-acid-concentration-increasing agent of the present invention and used to treat or prevent obesity or diabetes (hereinafter also referred to as "the pharmaceutical composition of the present invention") has less concern about side effects and can be preferably used as pharmaceuticals suitable for continuous administration.

The form of the pharmaceutical composition of the present invention is not particularly limited, but the pharmaceutical composition of the present invention is preferably a pharmaceutical composition for oral administration from the viewpoint of easy continuous ingestion. The pharmaceutical composition of the present invention may be in any of solid, liquid and gel forms.

Examples of the form of the pharmaceutical composition for oral administration include formulations such as capsules, tablets, pills, powders, fine granules, granules, liquid medicines and syrups.

The pharmaceutical composition of the present invention is preferably a composition including the blood-decanoic-acid-concentration-increasing agent of the present invention and pharmacologically and pharmaceutically acceptable additives. As the "pharmacologically and pharmaceutically acceptable additives" commonly a substance which is used regularly as e.g. an excipient in the pharmaceutical field, and does not react to the active ingredient (i.e. DL2 and/or D2L) included in the blood-decanoic-acid-concentration-increasing agent of the present invention, can be used.

The dosage of the pharmaceutical composition of the present invention can be appropriately set depending on administration purposes (prevention or treatment), administration methods, administration period, and other conditions (e.g. the symptom, age and body weight of patients). In the case of oral administration, the lower limit value of the dosage of the pharmaceutical composition of the present invention can be set to preferably 1 mg/kg body weight/day or more, and further preferably 5 mg/kg body weight/day or more in terms of the amount of decanoic acid in the pharmaceutical composition of the present invention. The upper limit value can be set to preferably 2500 mg/kg body weight/day or less, and further preferably 1000 mg/kg body weight/day or less in terms of the amount of decanoic acid in the pharmaceutical composition of the present invention.

The pharmaceutical composition of the present invention has less concern about side effects, and moreover has a low possibility of interaction with common active ingredients, and thus may be used in combination with an existing drug (e.g. anti-obesity drug, antidiabetic drug). When the existing drug combined with the pharmaceutical composition of the present invention is an antidiabetic drug, it is thought that the dose of the existing drug can be reduced, and thus there is a possibility that the side effects of the existing drug can be reduced.

The pharmaceutical composition of the present invention has less concern about side effects, and thus is suitable for continuous administration. The administration period is not particularly limited, and when symptom improvement is observed in the prevention and treatment of diabetes or obesity, continuous ingestion is preferred. The composition may be administered every several hours or at intervals (e.g. a day to several days) in the above period.

### [Food composition]

The blood-decanoic-acid-concentration-increasing agent of the present invention can be applied to produce a food composition used for suppression of postprandial elevation of blood glucose level, slim body shape, obesity prevention or obesity improvement. The triglyceride (DL2 and/or D2L), an active ingredient, of the blood-decanoic-acid-concentration-increasing agent of the present invention not only has less concern about side effects, but also does not easily lose the flavor and palatability of food. Therefore, the food composition including the blood-decanoic-acid-concentration-increasing agent of the present invention and used for suppression of postprandial elevation of blood glucose level, slim body shape, obesity prevention or obesity improvement (hereinafter also referred to as "the food composition of the present invention") can be preferably used as food which is easily eaten.

Examples of the form of the food composition of the present invention include supplements, general food, food for animals and animal feed. In addition, the food composition of the present invention may be in any of solid, liquid and gel forms.

The form of the supplements is not particularly limited, and may be a solid formulation or a liquid formulation. Examples thereof can include formulations such as tablets, coated tablets, capsules, granules, powders, powdered medicine, sustained release formulations, suspension, emulsion, oral solutions, sugar coated tablets, pills, fine granules, syrups and elixirs.

The form of the general food is not particularly limited, and examples thereof can include bakery and confectionery (e.g. bread, cake, cookies, biscuits, doughnuts, muffins, scones, chocolate, snacks, whipped cream, ice cream), beverages (e.g. fruit juice drinks, energy drinks, sports drinks), soup, flavored processed food (e.g. dressing, sauce, mayonnaise, butter, margarine, prepared margarine), fatspread, shortening, bakery mix, frying oil, deep-frying oil, deep-fried food, processed meat products, frozen food, deep-fried food, noodles, retort pouched food, liquid food, food for dysphagia and the like.

When the blood-decanoic-acid-concentration-increasing agent of the present invention is used to produce general food, it is preferred that the triglyceride (DL2 and/or D2L), an active ingredient, of the blood-decanoic-acid-concentration-increasing agent of the present invention be directly added to raw materials or part of or all fats and oils, raw materials, be replaced with the triglyceride.

The triglyceride (DL2 and/or D2L), an active ingredient, of the blood-decanoic-acid-concentration-increasing agent of the present invention included in all triglycerides in the food composition of the present invention is preferably 20 mass% or more, more preferably 30 mass% or more, and yet further preferably 50 mass% or more. In addition, the upper limit value thereof is preferably 80 mass% or less, and further preferably 70 mass% or less. In the above range, the food composition of the present invention is effortlessly ingested like general food.

The amount of the food composition of the present invention ingested can be appropriately set depending on ingestion purposes (prevention or improvement), ingestion period and other conditions (e.g. the symptom, age and body weight of those who eat the composition). The lower limit value of the amount of the food composition of the present invention ingested can be set to preferably 1 mg/kg body weight/day or more, and further preferably 5 mg/kg body weight/day or more in terms of the amount of decanoic acid in the food composition of the present invention. The upper limit value can be set to preferably 2500 mg/kg body weight/day or less, and further preferably 1000 mg/kg body weight/day or less in terms of the amount of decanoic acid in the food composition of the present invention.

The food composition of the present invention may have indications stating that it is used for suppressing elevation of blood glucose level, or improvement in hyperglycemia conditions. The composition may have indications of, for example, "for those concerned about blood glucose level," "for those concerned about postprandial elevation of blood glucose level," "for those beginning to be concerned about blood glucose level," "to gently raise blood glucose level," "to gently absorb carbohydrate," "for those wanting to suppress rapid increases in blood glucose level" and "to reduce blood glucose level."

The food composition of the present invention may have indications stating that it is used for body fat reduction and/or obesity prevention or improvement by suppression of body fat accumulation. The composition may have indications of, for example, "for those concerned about body weight," "to enhance metabolism," "to help to reduce body fat of those slightly overweight," "to enhance fat metabolism," "to support good figures," "for those concerned about body fat," "for those slightly overweight," "for those concerned about body weight (BMI)," "to reduce body weight and abdominal fat (visceral fat and subcutaneous fat)," "to reduce waist circumference," "to become thin," "for ideal body," "for dieting" and "to become slim."

### EXAMPLES

The present invention will now be described in more detail by way of Examples and Comparative Examples. It should be noted, however, that the present invention is not restricted thereto in any way.

### [Preparation of triglyceride]

As described below, various triglycerides (TG1 to 4) were prepared. It should be noted that the triglyceride having as constituent fatty acids decanoic acid and a long chain fatty acid (i.e. the blood-decanoic-acid-concentration-increasing agent of the present invention) is TG2 and TG3. The amount of triglyceride molecular species in TG1 to 4 was measured using gas chromatography (JAOCS, vol 70, 11, 1111-1114 (1993) and the silver ion column-HPLC method (in accordance with J. High Resol. Chromatogr., 18, 105-107 (1995))). In addition, the fatty acid compositional ratios of various triglycerides were measured in accordance with "Standard Method for the Analysis of Fats, Oils and Related Materials 2.4.2.3-2013 fatty acid compositional ratio (capillary gas chromatography)" established by Japan Oil Chemists' Society. The measurement results were shown in Tables 1 and 2.

### [TG1]

A triglyceride having as constituent fatty acids only decanoic acid (tridecanoin: a product manufactured by The Nisshin Oillio Group Ltd.) was considered TG1.

### [TG2]

High oleic sunflower oil (product name: Nisshin himawari oil, manufactured by The Nissin Oillio Group Ltd.) and ethyl decanoate (manufactured by Tokyo Chemical Industry Co., Ltd.) were mixed in a mass ratio of 4:6. To the obtained mixture, an sn-1,3-position specific lipase (product name: Lipozyme RMIM, manufactured by Novozymes) was added so that the concentration was 0.3 mass% relative to the oil. While stirring the mixture in a reaction flask, an enzyme reaction was carried out at 50°C for 24 hours to obtain a reacted oil. The enzyme, the free fatty acids and the ester were removed from the reacted oil by conventionally methods to obtain a purified reacted oil. The purified reacted oil and the ethyl decanoate were mixed in a mass ratio of 2 : 8, and the same enzyme reaction as above and the same purification as above were carried out, and decolorization and deodorization were further carried out by conventional methods to obtain TG2.

### [TG3]

Tridecanoin (a product manufactured by The Nissin Oillio Group Ltd.) and ethyl oleate (manufactured by Wako Pure Chemical Industries, Ltd.) were mixed in a mass ratio of 8:2. To the obtained mixture, an sn-1,3-position specific lipase (product name: Lipozyme RMIM, manufactured by Novozymes) was added so that the concentration was 0.3 mass% relative to the oil. While stirring the mixture in a reaction flask, an enzyme reaction was carried out at 50°C for 10 hours to obtain a reacted oil. The enzyme, the free fatty acids and the ester were removed from the reacted oil by conventional methods, and decolorization and deodorization were further carried out by conventional methods to obtain TG3.

### [TG4]

Tridecanoin (a product manufactured by The Nissin Oillio Group Ltd.) and high oleic sunflower oil (product name: Nisshin himawari oil, manufactured by The Nissin Oillio Group Ltd., triglyceride amount 98 mass%) were mixed so that the amount of decanoic acid in all constituent fatty acids of triglyceride was equal to the amount of decanoic acid in TG2 to obtain TG4.

**[Table 1]**

| FATTY ACID COMPOSITIONAL RATIO OF TG 1 TO 4 (MASS%) | | |
|---|---|---|
| Triglyceride | n-Decanoic acid | Long chain fatty acids (C14-C22) |
| TG1 | 100.0 | 0.0 |
| TG2 | 53.7 | 46.3 |
| TG3 | 59.1 | 40.9 |
| TG4 | 53.7 | 46.3 |

The "C10-P-C10," "C10-C10-P," "C10-O-C10," "C10-C10-O," "C10-L-C10" and "C10-C10-L" in Table 2 correspond to D2L and the "C10-O-O" and "O-C10-O" correspond to DL2. In addition, "Others" correspond to LCT.

**[Table 2]**

| TRIGLYCERIDE MOLECULAR SPECIES COMPOSITIONAL RATIO OF TG 1 TO 4 (MASS%) | | | | |
|---|---|---|---|---|
| Molecular species | TG1 | TG2 | TG3 | TG4 |
| C10-C10-C10 | 100.0 | 14.0 | 14.3 | 53.7 |
| C10-P-C10 | 0.0 | 0.4 | 0.0 | 0.0 |
| C10-C10-P | 0.0 | 0.0 | 6.4 | 0.0 |
| C10-O-C10 | 0.0 | 69.9 | 0.0 | 0.0 |
| C10-C10-O | 0.0 | 0.0 | 60.5 | 0.0 |
| C10-L-C10 | 0.0 | 7.0 | 0.0 | 0.0 |
| C10-C10-L | 0.0 | 0.0 | 6.1 | 0.0 |
| C10-O-O | 0.0 | 7.5 | 0.0 | 0.0 |
| O-C10-O | 0.0 | 0.0 | 9.2 | 0.0 |
| Others (LCT) | 0.0 | 1.2 | 3.5 | 46.3 |
| Total | 100.0 | 100.0 | 100.0 | 100.0 |
| TOTAL AMOUNT OF D2L | 0.0 | 77.3 | 73.0 | 0.0 |
| TOTAL AMOUNT OF DL2 | 0.0 | 7.5 | 9.2 | 0.0 |

| | | | | |
|---|---|---|---|---|
| C10: n-Decanoic acid, P: Palmitic acid, O: Oleic acid, L: Linoleic acid | | | | |

### [Administration test of triglyceride to rat]

Male SD rats aged 7 weeks (Japan SLC, Inc.) were kept using a TPX cage (W21.8 x D37.3 X H18.1 cm) with three rats or less/cage. The rats were fed ad libitum with solid feed "Labo MR stock" (Nosan Corporation.) sterilized with radiation throughout the feeding period, and fasted about 16 hours before the administration of a test fat and oil sample. The rats were fed ad libitum with distilled water as drinking water using a water supply bottle. The acclimation period was a week, and it was confirmed that abnormalities of general conditions were not observed on the final day of the acclimation period, and body weight was measured using an electronic scale (GX-3000; A & D Company, Limited). The rats were divided into 6 groups for each test fat and oil sample using a grouping software (Statlight; Yukms Co., Ltd.) so that the body weight of the groups was almost equal (6 rats in each group) .

Each TG was orally administered in a single dose at 1 mL/body using a 1 mL disposable syringe and a disposable gastric tube for rats. Blood was collected from the abdominal aorta under anesthesia with isoflurane inhalation an hour after administration. The blood was put in a serum separator tube (BD Vacutainer blood collection tube, micronized silica particles to accelerate clotting/serum separator; Nippon Becton Dickinson Company, Ltd.) and allowed to stand at room temperature for 30 minutes or more, and then centrifuged at 3,000 rpm for 15 minutes using a floor refrigerated centrifuge (H-60R; KOKUSAN Co., Ltd.) to obtain a serum.

To a glass test tube, chloroform/methanol/serum = 1:2:0.8 (v/v/v) was added, and the obtained mixture was stirred well by a touch mixer. The mixture was allowed to stand with shading at room temperature for 10 minutes, and chloroform and distilled water were added thereto so that chloroform/methanol/serum + water = 1:1:0.9 (v/v/v) was obtained. The obtained mixture was stirred by a touch mixer, and then centrifuged (4°C, 1,500 rpm, 10 min). The lower layer, the chloroform layer, was collected with Pasteur pipette, and transferred to a glass test tube (NICHIDEN-RIKA GLASS CO., LTD.). To the remaining water layer, chloroform was added in the same amount as collected, and the obtained mixture was stirred by a touch mixer and then similarly centrifuged to collect the lower layer. The collected chloroform layer was dried up to obtain the total lipid fraction.

To the extracted lipid fraction, 2 ml of a 0.5 mol/L NaOH-methanol solution was added. The obtained mixture was stirred well by a touch mixer, and allowed to stand in a 100°C block heater for 7 minutes. The mixture was allowed to cool to room temperature, and 3 mL of a boron trifluoride methanol complex solution (Sigma-Aldrich) was then added thereto. The obtained mixture was stirred well by a touch mixer, and allowed to stand in a 100°C block heater for 5 minutes. The mixture was allowed to cool to room temperature, and 0.8 mL of hexane was then added thereto, and the obtained mixture was vigorously stirred. To the mixture, 7 mL of a saturated saline solution was added, and the obtained mixture was vigorously stirred. The hexane layer was transferred to a vial, and used for GC (Agilent Technologies) in the conditions shown in Table 3, and the blood decanoic acid concentration was determined by the internal standard method. The measurement results were shown in Table 4 and Fig. 1.

For differences in the blood decanoic acid concentration in the TG1 to 4 administered groups, the multiple comparison test was carried out using the Tukey-Kramer's Test method, and a level of significance of less than 5% (p < 0.05) was considered statistically significant. The results were shown in Fig. 1.

**[Table 3]**

| | |
|---|---|
| Column | TC-70 |
| Detector | FID |
| CT | 100°C∼(3°C/min)∼250°C |
| I/D | 250°C/260°C |
| Inlet pressure | 30psi |
| Injection amount | 1µL |

**[Table 4]**

| Administered triglyceride | Blood decanoic acid concentration | |
|---|---|---|
| | Average value(µM) | Standard error |
| TG1 | 124.4 | 15.8 |
| TG2 | 1208.8 | 146.7 |
| TG3 | 1216.9 | 271.4 |
| TG4 | 492.0 | 29.8 |

From the above results, each significant difference was verified in the TG1 administered group, TG2 or TG3 administered group, and TG4 administered group (Fig. 1). In addition, in the TG2 or TG3 administered group, the blood decanoic acid concentration was increased 9 times or more higher than that in the TG1 administered group, and the blood decanoic acid concentration was increased about 2.5 times higher than that in the TG4 administered group. Therefore, the blood decanoic acid concentration can be effectively increased by ingestion of a triglyceride, having as constituent fatty acids decanoic acid and a long chain fatty acid, such as TG2 and TG3, and thus the effects of e.g. slim body shape, obesity prevention or improvement, suppression of postprandial elevation of blood glucose level, and diabetes treatment or prevention could be expected.

## Claims

1. A method for increasing a blood decanoic acid concentration, comprising ingesting a triglyceride having decanoic acid and a long chain fatty acid as constituent fatty acids.

2. The method for increasing a blood decanoic acid concentration according to claim 1, wherein the triglyceride comprises a triglyceride in which two of the constituent fatty acids are decanoic acid and one is a long chain fatty acid, in a content of 50 mass% or more.

3. The method for increasing a blood decanoic acid concentration according to claim 1 or 2, wherein the long chain fatty acid is an unsaturated fatty acid.

4. The method for increasing a blood decanoic acid concentration according to claim 3, wherein the unsaturated fatty acid is oleic acid.

5. The method for increasing a blood decanoic acid concentration according to any one of claims 1 to 4, wherein slim body shape, prevention of obesity, or improvement in obesity is promoted by increasing the blood decanoic acid concentration.

6. The method for increasing a blood decanoic acid concentration according to any one of claims 1 to 4, wherein postprandial elevation of blood glucose level is suppressed and diabetes is treated or prevented by increasing a blood decanoic acid concentration.

7. A blood-decanoic-acid-concentration-increasing agent, comprising a triglyceride as an active ingredient, the triglyceride having decanoic acid and a long chain fatty acid as constituent fatty acids.

8. The blood-decanoic-acid-concentration-increasing agent according to claim 7, wherein the triglyceride comprises a triglyceride in which two of the constituent fatty acids are decanoic acid and one is a long chain fatty acid, in a content of 50 mass% or more.

9. The blood-decanoic-acid-concentration-increasing agent according to claim 7 or 8, wherein the long chain fatty acid is an unsaturated fatty acid.

10. The blood-decanoic-acid-concentration-increasing agent according to claim 9, wherein the unsaturated fatty acid is oleic acid.

11. A pharmaceutical composition for use in treatment or prevention of obesity or diabetes, the pharmaceutical composition comprising the blood-decanoic-acid-concentration-increasing agent according to any one of claims 7 to 10.

12. A food composition for use in any one of suppression of postprandial elevation of blood glucose level, slim body shape, obesity prevention or obesity improvement, the food composition comprising the blood-decanoic-acid-concentration-increasing agent according to any one of claims 7 to 10.

13. Use of a triglyceride having decanoic acid and a long chain fatty acid as constituent fatty acids for manufacturing a blood-decanoic-acid-concentration-increasing agent.
